# EUROPEAN PATENT APPLICATION

(11) **EP 2 223 615 A1**
(43) Date of publication of application: **01.09.2010**
(21) Application number: 09012445.4
(22) Date of filing: 01.10.2009
(51) Int. Cl.: A23L 1/275, A23G 9/42

(54) **Food grade colouring agent**

(30) Priority: 21.02.2009 EP 09002501
(71) Applicant: Rugeris, Jess Edward, West Molesey Surrey KT8 1QU (GB)
(72) Inventor: Rugeris, Jess Edward, West Molesey Surrey KT8 1QU (GB)
(74) Representative: Schaad, Balass, Menzl & Partner AG

(57) **Abstract**

The present invention relates to a food grade colouring agent for the preparation of coloured ice cubes, wherein the colouring agent comprises dried red cabbage, a method for the preparation of the colouring agent, comprising the steps of chopping and drying red cabbage, to a method for the preparation of a coloured liquid, wherein the colouring agent is extracted with water and/or steam, and to the use of the coloured liquid for the preparation for coloured ice cubes or confectionary.

## Description

The present invention relates to a food grade colouring agent, to a method for the preparation of the colouring agent, and to its use for the preparation of coloured ice cubes or confectionary.

Nowadays, a wide range of food grade colouring agents is used by the food industry in order to enhance the optical appearance of food.

Of the known food grade colouring agents, only a few are natural, such as betanoin or chlorophyll, for instance. Most food grade colouring agents are either synthetic analogs of naturally occurring substances - so-called nature identical substances - or are fully artificial.

The use of some of the artificial and synthetic colouring agents, e.g. of the azo dyes, for colouring food is highly controversial, as they are known or suspected to be allergenic, carcinogenic, or to have other undesired side effects on the human body or the environment.

It is therefore a problem of the present invention to provide food grade colouring agents in a wide range of colours, which are absolutely safe to use.

With respect to the preparation of coloured ice cubes and confectionary, for instance, it is particularly desirable not only to provide a wide range of colours, but also to avoid undesired side effects of such a colouring agent:
- It is desirable that coloured ice cubes can be used with essentially any kind of drink and coloured confectionary materials for any kind of food. Thus, in order to avoid unpleasant taste or undesired mixing of tastes, the coloured ice cubes or confectionary should be taste- and odourless.
- From a visual point of view, coloured ice cubes should not change the colour of the drink, for the cooling of which they are used.
- Since the coloured ice cubes or confectionary materials may by brought into contact with textiles, for instance if a drink is spilled on clothes, it is further desirable that the colouring agent, and thus the coloured ice cubes or confectionary, does not stain textiles, i.e. that it is "non-staining".

It is therefore a further problem of the present invention to provide food grade colouring agents which is "non-staining" and does not have a taste or smell.

The problem is solved by the colouring agent according to claim 1 and the methods according to claims 12 and 14. Preferred embodiments are subject of the dependent claims.

The present invention provides a food grade colouring agent, which comprises dried red cabbage.

The colouring agent of the present invention is very potent. As it is derived from red cabbage, i.e. a vegetable material, it is absolutely safe for use in food, drinks, and other products, which are applied to the human body, such as pharmaceutical or cosmetic products, for instance, and in particular for the reparation of coloured ice cubes or coloured confectionary. In order to obtain a particularly safe colouring agent, it is further possible to use organic red cabbage.

The colouring agent of the present invention has essentially no taste. In general, a tasteless substance will also be odourless. In the food industry and, in particular, in the beverage and confectionary industries, the use of colouring agents is common practice. Thanks to the colouring agent of the present invention, it is possible to conserve the original smell and taste of the food or drink. Therefore, the colouring agent of the present invention is particularly well suited for the preparation of coloured drinks, ice cubes or confectionary.

The colouring agent according to the present invention comprises at least dried red cabbage. Colouring agents comprising dried red cabbage offer a wide range of colours: Depending on the parameters used for the preparation of the colouring agent, such as the drying conditions, and on the particle size of the colouring agent, various shades of blue, turquoise, and purple can be obtained. In addition, it is possible to add other vegetable materials, such as dried lemon or saffron, in order to obtain an even wider range of colour, including various shades of green, orange, red, and pink.

Preferably, the colouring agent of the present invention does not comprise any synthetic dyes or other, non-natural additives. In a preferred embodiment, the food grade colouring agent of the present invention contains only dried red cabbage, which is preferably in granulate or powder form.

By using dried red cabbage, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried red cabbage.

From a colouring agent, which comprises at least dried red cabbage, the colour can easily be extracted using water or steam.

A further advantage of the colouring agent according to the present invention is that it is "non-staining": If a liquid coloured with the colouring agent is poured through a white cotton cloth, for instance, the cloth is not stained and remains white.

The colouring agent of the present invention comprises dried red cabbage. By adjusting the drying conditions, such as temperature, drying method or drying time, a colouring agent in a predetermined colour is obtained. A person skilled in the art is able to determine the exact conditions using pertinent preliminary tests.

In a preferred embodiment, the colouring agent comprise air or freeze dried red cabbage. Alternatively, the red cabbage can also be spray dried. These drying methods allow for a fast and gentle drying of the red cabbage, affording the colouring agent in the desired colour and with a high colouring strength.

Air drying is particularly favourable for drying the red cabbage, as it allows preserving the desired colours while most of the aroma is removed. In addition, the colouring agent prepared by air drying is particularly stable. Even after several months of storage under air, no oxidation is observed. Since colouring agents comprising freeze dried red cabbage are somewhat less stable, they are preferably stored under vacuum to prevent oxidation.

In a preferred embodiment, the food grade colouring agent of the present invention contains only dried red cabbage, which is preferably in granulate or powder form.

In a preferred embodiment of the present invention, the colouring agent is in powder form. Such a powder is obtained by drying and subsequent grinding of the dried red cabbage. Due to the relatively large surface area of the individual particles, a colouring agent in powder form is particularly potent and the colour can be very easily extracted therefrom. In addition, a red cabbage based colouring agent in powder form yields a very particular range of colours.

In an alternative preferred embodiment, the colouring agent is a granulate. The colouring agent granulate is obtained by chopping the red cabbage into pieces of about 2 to 4 cm before drying. Compared to the powder, the granulate is more stable towards oxidation. If the colouring agent of the present invention is provided as a granulate, it affords a particular range of colours, which is different from the one observed for the corresponding powder.

In a preferred embodiment, at least the colour of the colouring agent is soluble in water and/or steam. This allows for extraction of the colour using water and/or steam immediately before the introduction into the product to be coloured. Preferably, only the colour is soluble in water and/or steam, whereas the rest of the colouring agent is not soluble and can easily be removed from the water or steam by filtration after use.

More preferably, the colour of the colouring agent is also soluble in cold water, which is the case if the colouring agent of the present invention is supplied in powder form. Preferably, the colour of the colouring agent can be extracted with cold water having a temperature of less than 30°C, more preferably of less than 20 °C, and most preferably in the range of 5 °C to 15 °C.

In a preferred embodiment of the present invention, the colouring agent is in a gas and/or liquid permeable package, preferably a sachet or pod. Inserting the dried red cabbage into such a package offers a wide range of advantages: The colouring agent can be allotted in the desired amounts into each package to provide a single dose. Several single dose packages can then be further packaged into a container suitable for storage, transport and sale of the colouring agent. When using the colouring agent, it is not necessary to touch the dried red cabbage directly, but only the surrounding package, thus avoiding contamination. Furthermore, during the actual colouring process, the gas and/or liquid permeable package containing the food grade colouring agent can be inserted into a liquid or gas, which is able to extract the colour. The colour itself is "washed out" of the package by the liquid or gas, whereas the solid residues of the colouring agent are kept inside the package and can easily be removed from the extraction liquid or gas.

In a preferred embodiment, the colouring agent of the present invention provides a blue colour, which is preferably in the range of the Pantone colours No. 072, 285, 286, 292, 293, 297, 299, 300, 306, 631, and 801. Such a blue colouring agent is obtainable by chopping and drying red cabbage for 7 to 11 hours at about 50 °C to 55 °C in order to obtain a granulate. The blue colouring agent is very potent and affords a coloured liquid of a very brilliant blue. In addition, thanks to the method of preparation used for the colouring agent of the present invention, the colouring agent is "non-staining", whereas red cabbage itself does very well stain textiles and similar materials.

In another preferred embodiment, the colouring agent of the present invention provides a purple colour, which is preferably in the range of the Pantone colours No. 527 and 528. Such a purple colouring agent is by chopping and drying red cabbage for 7 to 11 hours at about 50 °C to 55 °C, followed by grinding of the dried red cabbage to obtain a powder. The purple colouring agent is very potent and affords a coloured liquid of a very brilliant purple. In addition, thanks to the method of preparation used for the colouring agent of the present invention, the colouring agent is "non-staining", whereas red cabbage itself does very well stain textiles and similar materials.

In another preferred embodiment, the colouring agent of the present invention provides a turquoise colour, which is preferably in the range of the Pantone colours No. 333, 335, 377, 3405, 346, and 354. A turquoise colour is obtainable by extracting about 1 to 2 g of the above described granulate with about 10 to 20 ml of hot water and applying it to sugar. Upon heating of the sugar to > 102 °C, the colour changes from sapphire blue to turquoise shades. The turquoise colouring agent is very potent and affords confectionary of a very brilliant turquoise blue or turquoise green.

Further preferred colours obtainable with the colouring agent of the present invention include shades of red and pink, for instance in the range of the Pantone colours No. 185 and 1925 (ruby and rhodamine red). Red and pink shades can be obtained by adding a few drops of lemon juice to the dried red cabbage.

In a further aspect, the present invention also relates to a method for the preparation of the colouring agent of the present invention. Said method comprises the steps of chopping and drying red cabbage. The colouring agent thus obtained is generally in granulate form. Optionally, depending on the desired colour, the dried red cabbage is ground to afford the colouring agent in powder form.

The method of the present invention allows for a very straight forward, cost effective preparation of the colouring agent. The method of the present invention affords a potent colouring agent, which is derived from red cabbage. It is therefore absolutely safe for use in food, drinks, and other products, which are applied to the human body, such as pharmaceutical or cosmetic products. In order to obtain a particularly safe colouring agent, it is possible to use organic vegetable materials.

In order to facilitate the drying process, the red cabbage is cut (chopped) or ground before being dried. In this manner, the surface area of the red cabbage is enhanced and the drying process accelerated. The method of this preferred embodiment affords the colouring agent in relatively small particles having a large surface area, which can easily be measured and dosed. Furthermore, the enlarged surface area also improves the colouring agent's colouring properties, since the colour is released through the surface.

The red cabbage is dried under conditions resulting in a predetermined colour. By drying the red cabbage, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried vegetable material. At the same time, the method of the present invention allows for the preparation of a food grade colouring agent having a predetermined colour by adjustment of the drying conditions, such as temperature, drying method or drying time. A person skilled in the art is able to determine the exact conditions using pertinent preliminary tests.

In a preferred embodiment, the red cabbage is air or freeze dried. Alternatively, the red cabbage can also be spray dried. These drying methods allow for a fast and gentle drying of the vegetable materials, affording the colouring agent in the desired colour and with a high colouring strength.

Air drying is particularly favourable for drying the red cabbage, as it allows preserving the desired colours while most of the aroma is removed. In addition, the colouring agent prepared by air drying is particularly stable. Even after several months of storage under air, no oxidation is observed.

By chopping the red cabbage into pieces of about 2 to 4 cm and drying the cut red cabbage, a colouring agent in granulate form is obtained. The granulate is more stable towards oxidation than a powder, and the extraction of the granulate affords a different range of colours than the extraction of the powder.

In order to obtain a colouring agent in powder form, on the other hand, the dried red cabbage is ground. Thanks to the grinding, the surface area of the particles of the colouring agent is maximized, thus facilitating the release of colour from the colouring agent. Surprisingly, it has been found that it is even possible to extract the colour from the colouring agent with cold water, if the colouring agent is in powder form, whereas hot water or steam is necessary in the case of a colouring agent in granulate form.

As has been described above, the colour range of the colouring agent varies with its particle size, the powder affording purple-blue shades and the granulate affording blue or turquoise shades.

In a preferred embodiment, the dried red cabbage is mixed with one or more other coloured vegetable materials, which have preferably also been dried. For instance, the dried red cabbage can be mixed with dried lemon zest or saffron.
By mixing several dried vegetable materials, it is possible to prepare food grade colouring agents in a large variety of different colours and in various shades. Thus, the colour can be adjusted to the needs of the industry and the desires of the consumers. Alternatively, it is also possible that one or more coloured vegetable materials are mixed with the red cabbage prior to drying.

In a further aspect, the present invention also relates to the use of the food grade colouring agent for the preparation of a coloured liquid by extracting the colouring agent with water or steam. By extracting the colouring agent of the present invention with water or steam, the colour is dissolved in the water or steam and - in the case of steam, after condensation - a coloured liquid is obtained.

In particular, the present invention also features a method for the preparation of a coloured liquid, wherein the colouring agent of the present invention is extracted with water and/or steam to afford the coloured liquid. This method allows for a very simple, fast, and straightforward preparation of a coloured liquid.

In a preferred embodiment, the coloured liquid is prepared by extracting the food grade colouring agent with cold water. This is possible if the colouring agent used is in powder form. Preferably, the cold water used for extraction has a temperature of less than 30 °C, more preferably of less than 20 °C, and most preferably in the range of 5 °C to 15 °C. The use of cold water for the extraction of the food grade colouring agent of the present invention is not only advantageous from an energetic point of view, as it saves the energy necessary for heating, but it also further facilitates the preparation of an odourless and tasteless coloured liquid.

If the coloured liquid is prepared by extracting the food grade colouring agent with hot water, which is necessary in case the colouring agent is in granulate form, the water used for extraction has preferably a temperature of at least 45 °C, more preferably of at least 70 °C. At a water temperature of more than 70 °C, pathogens potentially contained in the water are killed.

Alternatively, it is also possible to use the food grade colouring agent of the present invention for colouring a non-water-based liquid.

The coloured liquid obtained by extraction of the food grade colouring agent of the present invention is preferably used for the preparation of coloured ice cubes, coloured drinks or coloured food, such as confectionary. Alternatively, it can also be used to prepare coloured pharmaceutical or cosmetic products. In all these cases, the characteristics of the colouring agent of the present invention described above - namely simple and cost effective preparation, availability of a wide range of colours in different shades, and complete safety for human and animal beings - are particularly advantageous.

In order to prepare coloured ice cubes, the coloured liquid is frozen and brought into the desired shape by the typical methods for the preparation of ice cubes commonly used. The coloured ice cubes thus obtained are very colourful and bright. Furthermore, the colouring agents of the present invention allow for the preparation of ice cubes in a wide range of colours, from green to turquoise and blue and to purple, pink, red, and orange.

For the preparation of coloured confectionary, sugar is added to the coloured liquid and heated. In order to prepare turquoise coloured confectionary, sugar is boiled with blue water. In order to prepare red or purple coloured confectionary, sugar is boiled with blue water and a few drops of lemon juice. In the later case, a syrupy confectionary material is obtained, whereas the turquoise coloured confectionary material becomes solid after cooling.

### Examples

### Drying apparatus

The coloured vegetable materials were in general air dried, using a 28x28x76 cm cardboard box with a drying rack in the closed area, an internal 60 W light bulb, and breathing vents on top. The coloured vegetable material was placed on the drying rack and the light bulb was used to create dry heat.

### Example 1: Preparation of a blue food grade colouring agent

500 g of fresh, raw red cabbage leaves were cut into pieces of about 2 to 4 cm and air dried using the drying apparatus described above at a drying temperature of about 52 °C over a period of 7 to 11 hours to afford 50 g of dried red cabbage in granulate form. The dried red cabbage granulate was then, in an amount of 1 g each, inserted into sachets. One sachet was used to produce about 8-9 1 of deep blue water by extraction with hot water having a temperature of 70 to 100 °C. The blue water was used for the preparation of blue ice cubes. The blue colour typically observed is in the range of the Pantone colours No. 072, 285, 286, 292, 293, 297, 299, 300, 306, 631, and 801.

### Example 2: Preparation of a green food grade colouring agent

1 g of dried red cabbage in granulate form, which was obtained by the method described in example 1, was mixed in a ratio of 5:1 with dried saffron. The mixture was dipped into hot water having a temperature of 70 °C for 1 second and then allowed to air dry at a temperature of 35 °C over a period of 24 hours. The dried mixture was inserted into a sachet, which was used to produce up to 9 1 of lime green water by extraction with hot water having a temperature of 70 to 100 °C. The lime green water was used for the preparation of lime green ice cubes. The blue colour typically observed is in the range of the Pantone colours No. 368 and 375.

### Example 3: Preparation of a pink food grade coloring agent

1 g of dried red cabbage granulate, which was obtained by the method described in example 1, was soaked with 5 ml of lemon juice and was then allowed to air dry at a temperature of 35 °C over a period of 24 hours. The dried mixture was, in an amount of 1 g each, inserted into sachets. One sachet was used to produce up to 9 1 of pink water by extraction with hot water having a temperature of 70 to 100 °C. The pink water was used for the preparation of pink ice cubes. The pink colour typically observed is in the range of the Pantone colours No. 185 and 1925.

### Example 4: Preparation of an orange food grade colouring agent

1 g of dried red cabbage in granulate form, which was obtained by the method described in example 1, was mixed in a ratio of 5:1 1 witch dried saffron. 4 to 5 drops of lemon juice were added to the mixture and it was then allowed to air dry. The dried mixture was granulated and, in an amount of 1 g each, inserted into sachets. Each sachet can be used to produce up to 7 1 of orange or gold coloured water by extraction with hot water having a temperature of 70 to 100 °C. The orange water was used for the preparation of orange ice cubes.

### Example 5: Preparation of a turquoise confectionary material

The blue water obtained in example 1 was added to sugar and heated to > 100 °C to afford a turquoise confectionary material, which upon cooling becomes solid. The turquoise colour typically observed is in the range of the Pantone colours No. 333, 335, 377, 3405, 346, and 354.

### Example 6: Preparation of a red or pink confectionary material

The blue water obtained in example 1 and a few drops of lemon juice was added to sugar and heated to > 100 °C to afford a confectionary material in shades of red or pink, depending on the amount of lemon juice added. Upon cooling, the red or pink confectionary material stays liquid and adopts a syrupy texture. The red or pink colour typically observed is in the range of the Pantone colours No. 185 and 1925.

## Claims

1. Food grade colouring agent, wherein the colouring agent comprises dried red cabbage.

2. Colouring agent according to claim 1, comprising air dried red cabbage.

3. Colouring agent according to claim 1, comprising freeze dried red cabbage.

4. Colouring agent according to one of claims 1 to 3, wherein the colouring agent contains only dried red cabbage.

5. Colouring agent according to one of claims 1 to 4, wherein the colouring agent is in powder form.

6. Colouring agent according to one of claims 1 to 4, wherein the coloring agent is a granulate.

7. Colouring agent according to one of claims 1 to 6, wherein at least the colour of the colouring agent is soluble in water and/or steam, preferably in cold water.

8. Colouring agent according to one of claims 1 to 7, wherein the colouring agent is in a gas and/or liquid permeable package, preferably in a sachet or pod.

9. Colouring agent according to one of claims 1 to 8, wherein the colouring agent provides a blue colour, which is preferably in the range of the Pantone colors No. 072, 285, 286, 292, 293, 297, 299, 300, 306, 631, and 801.

10. Colouring agent according to one of claims 1 to 8, wherein the colouring agent provides a purple colour, which is preferably in the range of the Pantone colours No. 527 and 528.

11. Colouring agent according to one of claims 1 to 8, wherein the colouring agent provides a turquoise colour, which is preferably in the range of the Pantone colours No. 333, 335, 377, 3405, 346, and 354.

12. Method for the preparation of the colouring agent according to one of claims 1 to 1, comprising the steps of chopping and drying red cabbage.

13. Method according to claim 12, wherein the dried red cabbage is ground after the drying.

14. Method for the preparation of a coloured liquid, wherein the colouring agent according to one of claims 1 to 11 is extracted with water and/or steam to afford the coloured liquid.

15. Use of the coloured liquid of claim 14 for the preparation of coloured ice cubes or coloured confectionary.
